# EUROPEAN PATENT APPLICATION

(11) **EP 1 972 280 A1**
(43) Date of publication of application: **24.09.2008**
(21) Application number: 08005463.8
(22) Date of filing: 25.03.2008
(51) Int. Cl.: A61B 8/00

(54) **Ultrasound system and method for forming ultrasound images**

(30) Priority: 23.03.2007 KR 20070028400
(71) Applicant: MEDISON CO., LTD., Kangwon-do 250-870 (KR)
(72) Inventor: Choi, Won Seok, Seoul 135-280 (KR)
(74) Representative: Schmid, Wolfgang

(57) **Abstract**

The present invention is directed to an ultrasound system (100) and a method for displaying ultrasound images. The ultrasound system includes: a storage unit (150) operable to store batch processing information associated with a plurality of diagnostic modes in which the ultrasound system will consecutively operate in a batch mode; an input unit operable to allow a user to select the batch mode; a transmit/receive unit (110) responsive to the selection of the batch mode to consecutively transmit ultrasound signals to a target object and receive echo signals to thereby output reception signals in the respective diagnostic modes; a signal processing unit (120) operable to perform signal processing upon the reception signals based on the batch processing information for each of the diagnostic modes; an image processing unit (130) operable to form ultrasound images based on the signal-processed reception signals; and a display unit (140) operable to sequentially display the ultrasound images formed in the respective diagnostic modes.

## Description

The present application claims priority from Korean Patent Application No. 10-2007-0028400 filed on March 23, 2007, the entire subject matter of which is incorporated herein by reference.

### BACKGROUND OF THE INVENTION

### [Technical Field]

The present invention generally relates to an ultrasound system, and more particularly to an ultrasound system and a method of forming ultrasound images.

### [Background Art]

The ultrasound system has become an important and popular diagnostic tool due to its non-invasive and non-destructive nature. Modern high-performance ultrasound imaging diagnostic systems and techniques are commonly used to produce two- or three-dimensional images of internal features of patients.

An ultrasound system generally uses a probe containing an array of piezoelectric elements to transmit and receive ultrasound signals. The ultrasound system forms an image of human internal tissues by electrically exciting transducer elements to generate ultrasound signals that travel into the body. Echoes reflected from tissues and organs return to the transducer element and are converted into electrical signals, which are amplified and processed to produce ultrasound data. The ultrasound data may include volume data obtained by using a 3-dimensional probe, etc.

Generally, the ultrasound system is configured to provide ultrasound images of a target object in various diagnostic modes including a brightness (B) mode, a brightness/color (B/C) mode, a brightness/color (B/C) mode, a brightness/Doppler (B/D) mode and the like. For example, the ultrasound system may display a B-mode image of the target object based on reception signals received through a probe in the B-mode. Thereafter, if a user selects the B/C mode and sets a color box on the B-mode image, then the ultrasound system may form and display a color flow image within the color box. Also, if the user selects the B/D mode and sets a sample volume on the B-mode image, then the ultrasound system may form and display a Doppler spectrum image corresponding to the sample volume.

In the conventional ultrasound system, the diagnostic modes are manually selected one by one. Setup parameters such as gain, TGC and the like for image optimization should be set for each diagnostic mode. That is, the conventional ultrasound system may selectively operate in one of various diagnostic modes through a manual operation of the user. Thus, the conventional ultrasound system requires a complex manipulation by a user to change a diagnostic mode, thereby inconveniencing the user. Also, the long scanning time required for changing a diagnostic mode may be burdensome to a patient.

### BRIEF DESCRIPTION OF THE DRAWINGS

FIG. 1 is a block diagram showing an ultrasound system in accordance with one embodiment of the present invention.

FIGS. 2 and 3 are tables showing exemplary batch processing information for a heart and a liver, respectively, in accordance with the present invention.

FIG. 4 is schematic diagram showing an example of a display unit in which a plurality of screen areas is assigned thereon in accordance with the present invention.

FIG. 5 is a timing diagram showing an example of setting diagnostic periods in accordance with the present invention.

FIG. 6 is a schematic diagram showing an example of displaying various ultrasound images formed in various diagnostic modes in accordance with the present invention.

### DETAILED DESCRIPTION OF THE INVENTION

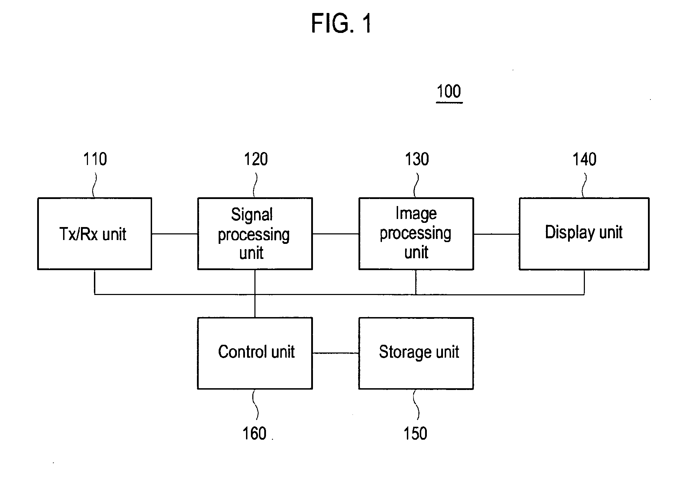

FIG. 1 is a block diagram showing an ultrasound system in accordance with one embodiment of the present invention. Referring to FIG. 1, the ultrasound system 100 includes a Transmit/Receive (T/R) unit 110, a signal processing unit 120, an image processing unit 130, a display unit 140, a storage unit 150 and a control unit 160. The control unit 160 may control entire operations of the elements of the ultrasound system 100. The ultrasound system 100 may further include an input unit for receiving various instructions from a user.

The T/R unit 110 may be operable to transmit ultrasound signals to a target object and receive echo signals of the transmitted ultrasound signals. The T/R unit 110 may output reception signals converted from the echo signals. The T/R unit 100 may transmit the ultrasound signals alternately in various diagnostic modes. The T/R unit 110 may include a probe for reciprocally converting ultrasound signals and electrical signals. The probe may comprise a plurality of transducer elements.

The signal processing unit 120 may be operable to focus the reception signals based on a distance between each transducer element and a focal point set in the target object and positions between the transducer elements. The signal processing unit 120 may perform signal processing (e.g., adjustment of gain, TGC, focal point position, the number of focal points, etc.) upon the reception signals.

The image processing unit 130 may be operable to form a reference image (e.g., B-mode image) and a plurality of ultrasound images (e.g., color flow image, Doppler image, M-mode image, etc.) formed in various diagnostic modes based on the reception signals. The display unit 140 may display the reference ultrasound image and the ultrasound images.

The storage unit 150 may store batch processing information necessary for operation in a batch mode in accordance with one embodiment of the present invention. The batch mode is to cause the ultrasound system 100 operate consecutively in two or more diagnostic modes without any input from the user. Various types of batch modes may be set in accordance with the present invention. The batch processing information may include information upon a target object, diagnostic modes covered by the batch mode, screen areas for displaying the ultrasound images formed in the respective diagnostic modes on a screen of the display unit 140, diagnostic periods representing time duration of the respective diagnostic modes, a plurality of setup parameters for adjusting the ultrasound image at each diagnostic mode, and values of the setup parameters.

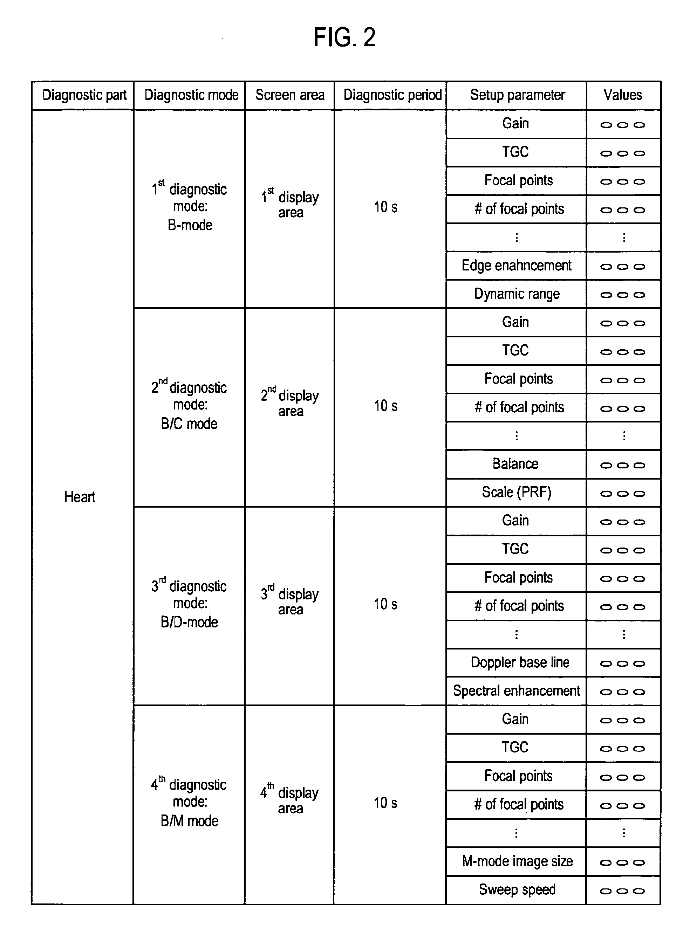

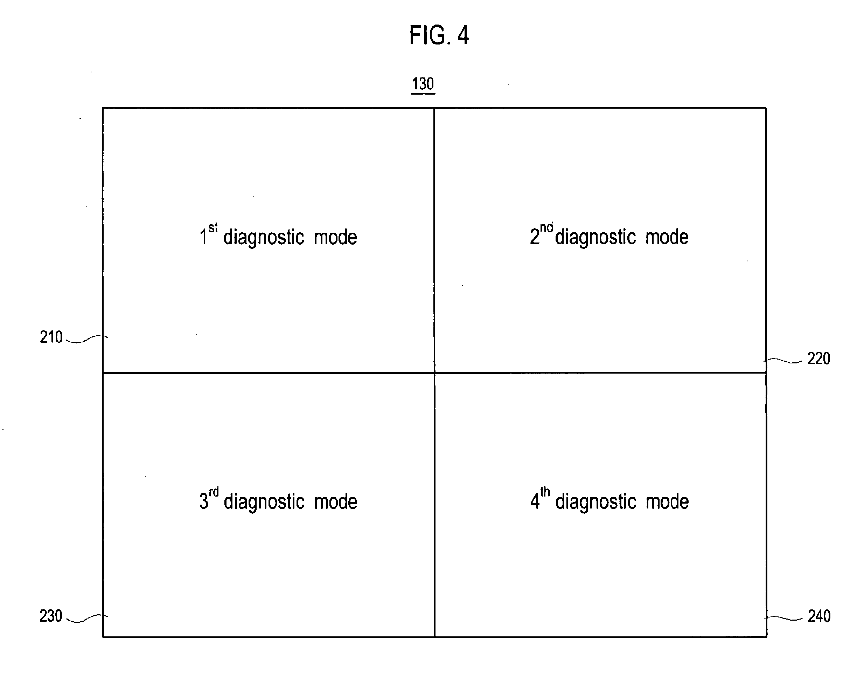

FIG. 2 is a table showing exemplary batch processing information for a heart as the target object in accordance with one embodiment of the present invention. As shown in FIG. 2, the diagnostic modes may include a brightness (B) mode, a brightness/color (B/C) mode, a brightness/Doppler (B/D) mode and a brightness/motion (B/M) mode. The screen areas may represent areas for displaying the ultrasound images formed in the respective diagnostic modes on the screen of the display unit 140, as shown in FIG. 4. The ultrasound images consecutively formed in the first diagnostic mode (e.g., B-mode), the second diagnostic mode (e.g., B/C mode), the third diagnostic mode (e.g., B/D mode) and the fourth diagnostic mode (e.g., B/M mode) may be sequentially displayed on the first to fourth screen areas 210 to 240 on the screen of the display unit 140.

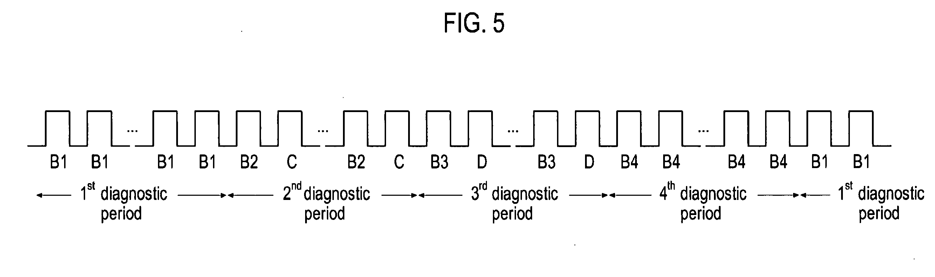

Diagnostic periods may represent the time duration necessary for forming and displaying an ultrasound image through transmission and reception of the ultrasound signals in each diagnostic mode. In FIG. 5, for example, the diagnostic periods may include first to fourth diagnostic periods during which ultrasound signals are transmitted so as to form and display the ultrasound image in the first to fourth diagnostic modes, respectively. The setup parameters may be used to adjust the ultrasound images in the respective diagnostic modes. For example, the gain parameter may be used to adjust gain of the reception signals outputted from the T/R unit 110 for adjusting brightness of the ultrasound image.

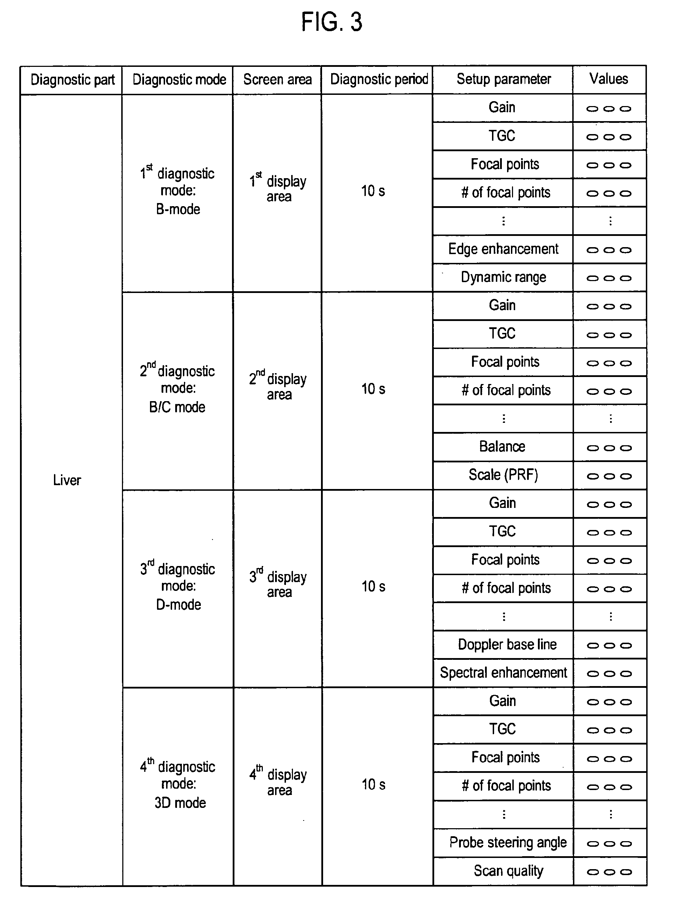

Although the batch processing information for the heart is described above in accordance with one embodiment of the present invention, it is obvious that the target object is not limited to the heart. FIG. 3 is a table showing exemplary batch processing information for a liver as the target object in accordance with another embodiment of the present invention. Also, the diagnostic modes, the screen areas, the diagnostic periods, the setup parameters and the setup values may be changed by a user in accordance with one embodiment of the present invention. Although the diagnostic modes are changed according to the diagnostic periods contained in the batch processing information in accordance with one embodiment of the present invention, the diagnostic modes may be changed in response to an input through the input unit from the user while the ultrasound system 100 operates in the batch mode in accordance with another embodiment of the present invention.

The control unit 160 may be operable to control entire operations of the elements such as formation and display of the ultrasound images in the diagnostic modes including the batch mode in the ultrasound system 100. The control unit 160 may include a period setting unit (not shown) for setting the diagnostic periods. For example, the period setting unit may include an electrocardiogram (ECG) trigger signal providing unit for providing an ECG signal in response to a heart beat. The diagnostic periods may be synchronized with the ECG signal.

The user input may receive setup instructions, which allow the user to set a color box, a sample volume, an M-line, a zoom box, a 3-dimensional box, etc. on the reference ultrasound image. If the user selects the batch mode and inputs the setup instructions through the input unit, then the control unit 160 may be operable to read out the batch processing information for the selected batch mode from the storage unit 150. When the processing information for various batch modes is stored in the storage unit 150, a list of the batch processing information may be displayed on the display unit 140 such that the user can select desirable batch processing information. The control unit 160 may be operable to control the elements of the ultrasound system 100 such that ultrasound images are sequentially formed and displayed based on the batch processing information in the respective diagnostic modes set in the batch mode.

Hereinafter, an example of an operation of the ultrasound system 100 in the batch mode will be described with reference to FIGS. 2 and 4-6. If the user selects one of the batch modes and sets the size and position of the color box, M-line and sample volume on reference image through the input unit, then the batch processing information corresponding to the selected batch mode is read out from the storage unit 150 under the control of the control unit 160. For sake of explanation, it is assumed below that the ultrasound system 100 operates in the order of B-mode, B/C mode, B/D mode and B/M mode in response to the selection of batch mode.

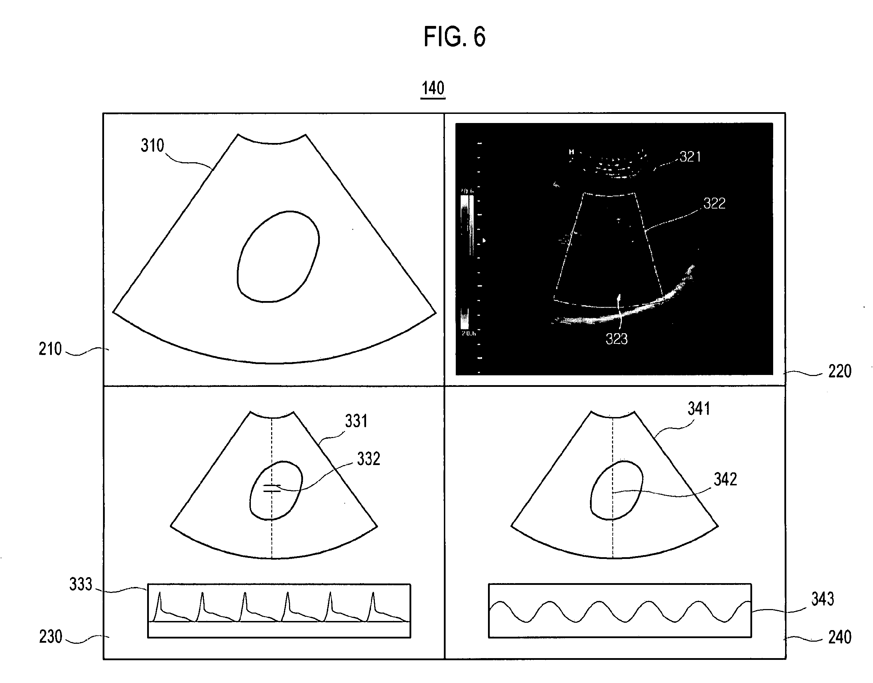

At a first diagnostic period, the ultrasound system 100 operates in the B-mode. That is, the T/R unit 100 transmits ultrasound signals B1 to the target object and forms the first reception signals based on echo signals of the ultrasound signals B1. The signal processing unit 120 may perform signal processing upon the first reception signals based on the corresponding batch processing information. The image processing unit 130 may form a B-mode image 310 based on the first reception signals. The display unit 140 may display the B-mode image on the first screen area 210, as shown in FIG. 6.

At the second diagnostic period, the ultrasound system 100 operates in the B/C mode. The T/R unit 100 repeatedly transmits ultrasound signals B2 and C for a B-mode image and a color flow image corresponding to the color box and outputs the second reception signals based on echo signals of the ultrasound signals B2 and C. The signal processing unit 120 may perform signal processing upon the second reception signals based on the corresponding batch processing information. The image processing unit 130 may form a B-mode image 321 and a color flow image 323 based on the second reception signals. The display unit 140 may display the B-mode image 321 and the color flow image 323 on the second screen area 220, as shown in FIG. 6.

At the third diagnostic period, the ultrasound system 100 operates in the B/D mode. The T/R unit 100 repeatedly transmits ultrasound signals B3 and D for a B-mode image and a Doppler image corresponding to the sample volume and outputs the third reception signals based on echo signals of the ultrasound signals B3 and D. The signal processing unit 120 may perform signal processing upon the third reception signals based on the corresponding batch processing information. The image processing unit 130 may form a B-mode image 331 and a Doppler image 333 based on the third reception signals. The display unit 140 may display the B-mode image 331 and the color flow image 333 on the second screen area 230, as shown in FIG. 6.

At the fourth diagnostic period, the ultrasound system operates in the B/M mode. The T/R unit 100 repeatedly transmits ultrasound signals B4 and M for a B-mode image and an M-mode image corresponding to the M-line and outputs the fourth reception signals based on echo signals of the ultrasound signals B4 and M. The signal processing unit 120 may perform the signal processing upon the fourth reception signals based on the corresponding batch processing information. The image processing unit 130 may form a B-mode image 341 and an M-mode image 343 based on the fourth reception signals. The display unit 140 may display the B-mode image 341 and the M-mode image 343 on the second screen area 240, as shown in FIG. 6.

The above process may be repeatedly carried out until an instruction for stopping the batch mode is inputted through the input unit. As mentioned above, since the various ultrasound images formed in the plurality of diagnostic modes can be consecutively formed in response to the selection of the batch mode, the examining time may be reduced.

In accordance with one embodiment of the present invention, there is provided an ultrasound system, including: a storage unit operable to store batch processing information associated with a plurality of diagnostic modes in which the ultrasound system will consecutively operate in a batch mode; an input unit operable to allow a user to select the batch mode; a transmit/receive unit responsive to the selection of the batch mode to consecutively transmit ultrasound signals to a target object and receive echo signals to thereby output reception signals in the respective diagnostic modes; a signal processing unit operable to perform signal processing upon the reception signals based on the batch processing information for each of the diagnostic modes; an image processing unit operable to form ultrasound images based on the signal-processed reception signals; and a display unit operable to sequentially display the ultrasound images formed in the respective diagnostic modes.

In accordance with another embodiment of the present invention, there is provided a method of displaying ultrasound images in an ultrasound system, comprising: storing batch processing information associated with a plurality of diagnostic modes in which the ultrasound system will consecutively operate in a batch mode; selecting the batch mode; consecutively transmitting ultrasound signals to a target object and receiving echo signals in response to the selection of the batch mode to thereby output reception signals in the respective diagnostic modes; performing signal processing upon the reception signals based on the batch processing information for each of the diagnostic modes; forming ultrasound images based on the signal-processed reception signals; and sequentially displaying the ultrasound images formed in each of the diagnostic modes.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system for displaying ultrasound images, comprising:
a storage unit operable to store batch processing information associated with a plurality of diagnostic modes in which the ultrasound system will consecutively operate in a batch mode;
an input unit operable to allow a user to select the batch mode;
a transmit/receive unit responsive to the selection of the batch mode to consecutively transmit ultrasound signals to a target object and receive echo signals to thereby output reception signals in the respective diagnostic modes;
a signal processing unit operable to perform signal processing upon the reception signals based on the batch processing information for each of the diagnostic modes;
an image processing unit operable to form ultrasound images based on the signal-processed reception signals; and
a display unit operable to sequentially display the ultrasound images formed in the respective diagnostic modes.

2. The ultrasound system of Claim 1, wherein the batch processing information includes information upon the target object, diagnostic modes covered by the batch mode, screen areas for displaying the ultrasound images on a screen of the display unit, diagnostic periods representing time duration for each of the diagnostic modes, a plurality of setup parameters for adjusting the ultrasound images at each diagnostic mode, and values of the setup parameters.

3. The ultrasound system of Claim 2, wherein the signal processing unit performs the signal processing upon the reception signals based on the setup parameters and the values.

4. The ultrasound system of Claim 1, wherein the input unit is further operable to receive setup instructions for allowing a user to set a color box, sample volume and an M-line on an ultrasound image and an input for changing the diagnostic modes while the ultrasound system operates in the batch mode.

5. A method of displaying ultrasound images in an ultrasound system, comprising:
storing batch processing information associated with a plurality of diagnostic modes in which the ultrasound system will consecutively operate in a batch mode;
selecting the batch mode;
consecutively transmitting ultrasound signals to a target object and receiving echo signals in response to the selection of the batch mode to thereby output reception signals in the respective diagnostic modes;
performing signal processing upon the reception signals based on the batch processing information for each of the diagnostic modes;
forming ultrasound images based on the signal-processed reception signals; and
sequentially displaying the ultrasound images formed in each of the diagnostic modes.

6. The ultrasound system of Claim 5, wherein the batch processing information includes information upon the target object, diagnostic modes covered by the batch mode, screen areas for displaying the ultrasound images on a screen of the display unit, diagnostic periods in the diagnostic mode, a plurality of setup parameters for adjusting the ultrasound images at each diagnostic mode, and values of the setup parameters.

7. The ultrasound system of Claim 6, wherein the signal processing is performed upon the reception signals based on the setup parameters and the values.

8. The ultrasound system of Claim 5, further comprising receiving setup instructions for allowing a user to set a color box, sample volume and an M-line on an ultrasound image.
